# EUROPEAN PATENT APPLICATION

(11) **EP 2 848 267 A1**
(43) Date of publication of application: **18.03.2015**
(21) Application number: 14184656.8
(22) Date of filing: 12.09.2014
(51) Int. Cl.: A61L 31/16

(54) **Anti-tumor macrophage m1 morphology inducer**

(30) Priority: 13.09.2013 US 201361877310 P
(71) Applicant: Cook Medical Technologies LLC, Bloomington, IN 47402 (US)
(72) Inventor: Gemborys, Colleen, Bloomington, IN 47403 (US)
(74) Representative: Simpson, Tobias Rutger

(57) **Abstract**

Described are embolization devices which carry M1 macrophage promoting agents and/or M2 macrophage inhibiting agents, as well as methods for their manufacture and use. An illustrative embolization device of the disclosure comprises an embolic body and one or more M1 macrophage promoting agents and/or M2 macrophage inhibiting agents carried by a surface of the embolic body. In certain embodiments the embolic body of the present disclosure comprises an embolic coil or an embolic bead.

## Description

### BACKGROUND

The present invention relates generally to medical devices and in particular aspects to embolization devices.

As further background, during the diagnosis, treatment, and follow-up of various medical conditions, it may be necessary or desirable for a physician to occlude a passageway or other open space within a patient's body. For example, cancer treatments often involve embolic therapies which restrict blood flow to the tumor in order to cut off the supply of nutrients and oxygen causing ischemia within the tumor. Embolic therapy has been indicated, for example, in the treatment of hepatocellular carcinoma, hepatic metastases from colorectal cancer and neuroendocrine tumors, and renal cell carcinoma.

A number of minimally invasive procedures have been developed whereby tumors can be treated using embolization devices. Such devices can include coils, beads, injectable "fillers," and various other implants. In some instances, one or more embolization devices are delivered to a tumor treatment site using a catheter (and possibly a guide-wire) that is advanced from the groin to the treatment site. An embolization device is then inserted through the catheter and into the vessel to be blocked. Such a procedure can be repeated until enough devices are "packed" into the vessel to fill it. In some instances, the embolic device induces thrombosis to occlude the vessel.

Despite proper embolization, tumors can promote angiogenesis and overcome embolization. A need therefore exists for embolic therapy products which inhibit angiogenesis and/or promote ischemia of an embolized tumor.

### SUMMARY

In certain aspects, the present invention provides embolic bodies which carry M1 macrophage promoting agents and/or M2 macrophage inhibiting agents. In accordance with some forms of the invention, such embolic bodies are configured to release M1 macrophage promoting agents and/or M2 macrophage inhibiting agents at or near a tumor site. Accordingly, in one embodiment, the present disclosure provides an embolic therapy device that includes an embolic body and one or more M1 macrophage promoting agents, M2 macrophage inhibiting agents, or a combination thereof carried by the embolic body. In accordance with certain inventive variants, the embolic therapy device further includes one or more coating layers carried by a surface of the embolic body. In some forms, the M1 macrophage promoting agents and/or M2 macrophage inhibiting agents are carried within a coating layer. In certain embodiments, the coating layer may be effective to release the M1 macrophage promoting agents and/or M2 macrophage inhibiting agents. In certain embodiments, the embolic body may, for example, be an embolic coil or an embolic bead. In one aspect, the embolic therapy device may further include an immune stimulating compound.

In another aspect, the present disclosure provides a method of forming a coated embolization device. Such method comprises coating a bioactive material on a surface of the embolic body; the bioactive material comprising one or more M1 macrophage promoting agents, M2 macrophage inhibiting agents, or a combination thereof. In certain embodiments, the embolic body may, for example, be an embolic coil or embolic bead.

In another aspect the present disclosure provides an embolization therapy device as described herein for use in a method of treating a patient, the method comprising implanting the embolization therapy device in the patient.

In yet another aspect the present disclosure provides an M1 macrophage promoting agent suitable for treating cancerous tumors, wherein said M1 macrophage promoting agent is carried on an embolic member. Also disclosed herein is the use of said agent for treating cancerous tumors.

In still another aspect the present disclosure provides an M2 macrophage inhibiting agent suitable for treating cancerous tumors, wherein said M2 macrophage inhibiting agent is carried on an embolic member. Also disclosed herein is the use of said agent for treating cancerous tumors.

Additional embodiments, as well as features and advantages of embodiments of the invention, will be apparent from the description herein.

### BRIEF DESCRIPTION OF THE DRAWINGS

FIG. **1** is a perspective view of an embolic therapy device according to one embodiment of the disclosure.
FIG. **2A** is a partial side view of another embolic therapy device of the disclosure.
FIG. **2B** is a partial enlarged view of the device of FIG. **2A****.**
FIG. **3A** is a perspective view of another embolic therapy device of the disclosure.
FIG. **3B** is a cutaway view of one embodiment of an embolic therapy device of the disclosure.
FIG. **4** is a cutaway view of vasculature surrounding a tumor.

### DESCRIPTION OF THE ILLUSTRATED EMBODIMENTS

While the present invention may be embodied in many different forms, for the purposes of promoting an understanding of the principles of the disclosure, reference will now be made to the embodiments illustrated in the drawings and specific language will be used to describe the same. It will nevertheless be understood that no limitation of the scope of the claims is thereby intended, and alterations and modifications in the illustrated device, and further applications of the principles of the disclosure as illustrated therein are herein contemplated as would normally occur to one skilled in the art to which the disclosure relates.

As described above, in certain aspects, the present invention provides embolic bodies which carry M1 macrophage promoting agents and/or M2 macrophage inhibiting agents. For example, an illustrative embolization device of the invention comprises an embolic body and a coating material a surface of the embolic body. The coating material includes M1 macrophage promoting agent(s), M2 macrophage inhibiting agent(s), or a combination thereof. Certain inventive variants include more than one agent. In certain embodiments, both an M1 macrophage promoting agent, and an M2 macrophage inhibiting agent are carried by the embolic body. Such an embolic therapy device, alone or in conjunction with one or more other suitable devices, can be used to occlude, or at least promote and/or facilitate occlusion of, a lumen or other open space within a patient's body. The embolic body can exhibit any suitable size, shape, and configuration, and can be formed with one or more of a variety of biocompatible materials. The invention also provides methods of forming and utilizing such embolic therapy devices, as well as medical products that include such devices enclosed within sterile packaging.

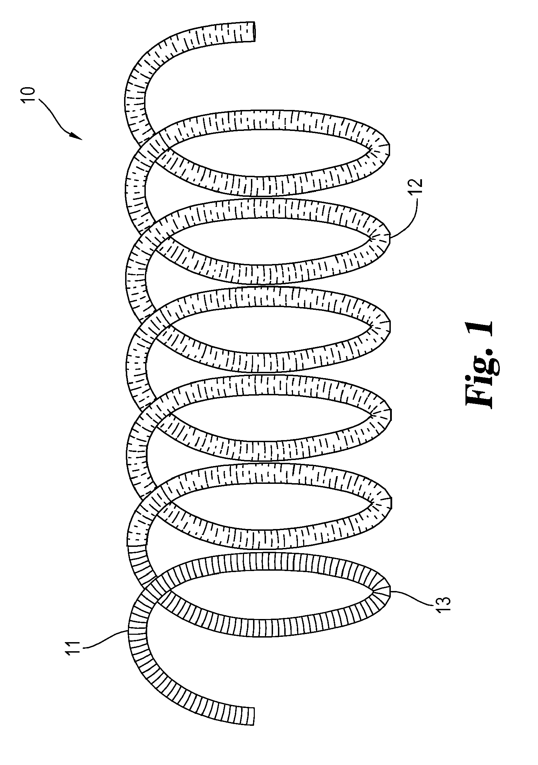

With reference now to FIG **1****,** shown is a perspective view of an embodiment of an illustrative embolic therapy device **10** of the disclosure. The illustrated embolic therapy device **10** includes an embolic body **11** and a coating layer **12** including one or more M1 macrophage promoting agents, M2 macrophage inhibiting agents, or a combination thereof on a surface of embolic body **11.** The embolic body **11** has an overall shape of a helical coil, although other shapes and configurations are envisioned. In some forms, embolic body **11** is formed of a biocompatible material such as platinum. As discussed more thoroughly below, such an overall helical coil shape can be formed by winding a length of wire into a primary coil, and then winding the primary coil into a secondary (helical) coil. A coating material such as that shown in FIG. **1** may be applied before and/or after formation of the primary and/or secondary coil, and may or may not coat the entire surface of the embolic body **11.** In the particular embodiment illustrated in FIG. **1** the embolic body **11** is shown having a coated portion **12** and an uncoated portion **13**.

The embolic therapy devices described herein may have a thrombogenicity and/or an occlusion inducing, promoting, and/or facilitating quality. In this regard, embolic therapy devices of the present disclosure can be used alone or in conjunction with one or more other suitable devices to occlude, or at least promote and/or facilitate occlusion of, a vessel or other open space within a patient's body.

For example, the embolic therapy device can be used to induce thrombus formation, which can lead to endothelialization, in a blood vessel surrounding a tumor. In certain forms, such occlusive qualities are enhanced by selecting coating materials that are receptive to tissue ingrowth, and in some cases, selecting coating materials that induce and/or promote patient cells to grow into the coating material. Remodelable coating materials may be used in this context to promote cellular growth within the coating material, which can, *inter alia,* help to anchor the device at the implantation site and provide occlusion.

In use, the embolic therapy device of the present disclosure is implanted in the vasculature immediately surrounding a tumor, for example through a cannulated device such as a catheter. The embolic body of the present disclosure is configured to occlude the vessel and optionally induce thrombus formation within the vessel. Embolic bodies of the present disclosure additionally carry certain therapeutic agents *(e.g.* M1 macrophage promoting agents and/or M2 macrophage inhibiting agents) which are effective to reduce angiogenesis at the tumor site as will be discussed herein. Importantly, the therapeutic agents may be carried in a coating layer, impregnated within the embolic body, or otherwise carried by the embolic body in any suitable manner.

Turning now to a discussion of M1 and M2 macrophage morphologies. Cancer is a result of abnormal and uninhibited cell growth. As stated above, embolization treatments are directed at restricting blood flow to cancerous tumors in order to restrict the tumor's supply of nutrients and oxygen. However, tumors can promote angiogenesis and overcome embolization. Tumor associated macrophages play an important role in a tumor's ability to promote angiogenesis. The tumor microenvironment is a dynamic multi-cellular environment in which macrophages may constitute up to 50% of the cells present. Macrophages originate from monocytes found in the blood stream and exhibit a substantial heterogeneity of phenotypes and specialization. For example, alternate morphologies have been found to be either immunostimulatory or immune suppressive, and either promote or restrain inflammation and angiogenesis.

Tumors may further avoid degradation by expressing CD47 transmembrane protein. CD47 is highly expressed on cancer cells as compared with normal cells. CD47 interacts with the ligand signal regulatory protein α (SIRP-α) on macrophages to initiate a signaling cascade that results in the inhibition of phagocytosis.

There are two distinct macrophage populations, M1 and M2. M1 macrophages, also called "classically activated" macrophages, are activated following stimulation with bacterial products and Th1 cytokines such as IFN-γ and lipopolysaccharides. M1 macrophages are involved in Th1 cell response to pathogens. M1 macrophages are immunostimulatory, exhibit enhanced microbicidal capacity, secrete high levels of proinflammatory cytokines, and increase concentrations of superoxide anions, oxygen radicals, and nitrogen radicals, which are important in early phase tissue repair. M1 macrophages tend to enhance tumor isolation and phagocytosis.

In some forms, the present disclosure includes M1 macrophage promoting agents which, in certain embodiments, stimulate the recruitment of M1 type macrophages, monocyte development into M1 macrophages, and/or the differentiation of M2 macrophages into M1 macrophages. M1 macrophage promoting agents may include, for example: histidine-rich glycoprotein (HRG), 17β-estradiol (E2), interferon-gamma (IFNγ), lipopolysaccharide (LPS), iron, iron-dextran, and/or any other compound known to one of ordinary skill in the art to promote M1 macrophage development and/or differentiation. In certain embodiments, M1 macrophage promoting agents comprise anti-CD47 blocking antibodies which increase phagocytosis of the target tumor. In accordance with certain embodiments, anti-CD47 blocking antibodies competitively bind CD47 receptors on the surface of a tumor. In some forms, anti-CD-47 blocking antibodies comprise engineered SIRP-α variants, for example a B6H12 variant.

M2 macrophages, also called "tumor associated macrophages", are activated by Th2 cytokines such as interleukin-4 (IL-4), interleukin-10 (IL-10), and interleukin-13 (IL-13), as well as colony stimulating factor 1 (CSF-1). Tumors secrete many of these molecules, and thus promote M2 recruitment and differentiation of monocytes in the tumor microenvironment. M2 macrophages dampen proinflammatory cytokine levels, secrete components of extracellular matrix essential for tissue repair, and support angiogenesis. These functions of M2 macrophages are important during later stages of wound healing; however, in the context of a growing tumor actually support disease progression.

In some forms, the present disclosure includes M2 macrophage inhibiting agents which inhibit the recruitment of M2 type macrophages, monocyte development into M2 macrophages, and/or the differentiation of M1 macrophages to M2 macrophages. In certain embodiments, such M2 macrophage inhibiting agents block, or inhibit, cytokines and other molecules secreted by tumors to recruit and activate M2 macrophages. M2 macrophage inhibiting agents may, for example, inhibit or block IL-4, IL-10, Il-13, CSF-1, and/or any other compound known to promote or induce M2 macrophages.

The size, shape, and configuration of the embolic therapy device of the present disclosure can vary. In some forms, embolic therapy products of the present disclosure are advantageously adapted to fit within the lumen of a suitable delivery device, either in a relaxed or unrelaxed condition, and then upon being deployed at the treatment site (e.g., within a lumen or other open space in the vasculature of an animal, preferably a mammal, even more preferably a human), to remain there and provide treatment to the patient. Suitable delivery devices include but are not limited to cannulated translumenally advanceable devices. In certain aspects, one or more devices are delivered to a treatment site, e.g., into the vasculature surrounding a tumor, using a catheter.

While the embolic body depicted in Figure 1 is formed with a biocompatible, metallic material, it should be noted that suitable embolic bodies can be formed with one or more of a variety of materials. These materials may be rigid, malleable, semi-flexible, or flexible. The material(s) selected for a particular embolic body can depend on a number of factors including, but not limited to, the intended use of the embolic therapy device, as well as its size, shape, and configuration. In general, suitable material(s) will be selected to allow a coated product of the invention to have certain desired performance and other characteristics, for example, to exhibit a flexibility falling within a desired range and/or to have shape memory.

Suitable biocompatible metallic materials that can be used in some forms of the invention include but are not limited to: gold, rhenium, platinum, palladium, rhodium, ruthenium, various stainless steels, tungsten, titanium, nickel, cobalt, molybdenum, manganese, iridium, silver, chromium, tantalum, iron, and copper, as well as alloys of these and other suitable metals, e.g., cobalt alloys, such as Elgiloy ®, a cobalt-chromium-nickel alloy, MP35N, a nickel-cobalt-chromium-molybdenum alloy, and a nickel-titanium alloy, e.g., Nitinol ®. In certain preferred aspects, an alloy is selected that exhibits excellent biocompatibility and yet has suitable strength and ductility to be wound into coils of primary, and potentially also secondary shape, and will retain any such shapes upon placement of the embolic therapy device in the body, particularly the human body. Additionally or alternatively, embolic bodies can include material in the form of yarns, fibers, and/or resins, e.g., monofilament yarns, high tenacity polyester, and the like, as well as other plastic, resin, polymer, woven, and fabric surgical materials, other conventional synthetic surgical materials, such as shape-memory plastics, and combinations of such materials. Further, one or more suitable ceramic materials including but not limited to hydroxyapatite, alumina and/or pyrolytic carbon can be used to form all or part of an embolic body.

Synthetic polymeric materials that can be used to form all or part of an embolic body include but are not limited to bioresorbable and non-bioresorbable plastics. Suitable bioresorbable, or bioabsorbable polymers include but are not limited to poly(L-lactic acid), polycaprolactone, poly(lactide-co-glycolide), poly(hydroxybutyrate), poly(hydroxybutyrate-co-valerate), polydioxanone, polyorthoester, polyanhydride, poly(glycolic acid), poly(D,L-lactic acid), poly(glycolic acid-co-trimethylene carbonate), polyhydroxyalkanaates, polyphosphoester, polyphosphoester urethane, poly(amino acids), cyanoacrylates, poly(trimethylene carbonate), poly(iminocarbonate), copoly(ether-esters) (e.g., PEO/PLA), polyalkylene oxalates, and/or polyphosphazenes. These or other bioresorbable materials may be used, for example, where only a temporary blocking or closure function is desired, and/or in combination with non-bioresorbable materials where only a temporary participation by the bioresorable material is desired.

Suitable non-bioresorbable, or biostable polymers include but are not limited to polytetrafluoroethylene (PTFE) (including expanded PTFE), polyethylene terephthalate (PET), polyurethanes, silicones, and polyesters and other polymers such as, but not limited to, polyolefins, polyisobutylene and ethylene-alphaolefin copolymers; acrylic polymers and copolymers, vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl aromatics, such as polystyrene, polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; alkyd resins, polycarbonates; polyoxymethylenes; polyimides; polyethers; epoxy resins, polyurethanes; rayon; and rayon-triacetate.

In some forms, biological materials such as extracellular matrix material may be used to form all or part of an embolic therapy device. For additional information as to some of the extracellular matrix materials useful in the present invention, and their isolation and treatment, reference can be made, for example, to U.S. Patent Nos. 4,902,508, 5,554,389, 5,993,844, 6,206,931, and 6,099,567, which are hereby incorporated by reference in their entirety. A suitable extracellular matrix material may be bioresorbable. In alternate embodiments a suitable extracellular matrix material may be cross-linked, and non-bioresorbable. Likewise, suitable extracellular matrix materials may or may not be expanded by contact with an alkaline substance. In certain embodiments, a suitable extracellular matrix material is compressed. Extracellular matrix materials suitable for the present disclosure may retain their native collagen structure or may be commutated and made flowable and castable.

While the embolic therapy device depicted in Figure 1 is generally in the shape of a helical coil, it should be understood that suitable embolic bodies can exhibit a variety of shapes. Advantageously, the shape selected will allow the device, by itself or in conjunction with one or more other suitable devices, to occlude, or at least promote and/or facilitate occlusion of, a space within a patient's body. Examples of suitable embolic bodies include but are not limited to, vascular occlusive devices including vaso-occlusive coils and microcoils, vascular wires, injectable embolic devices, embolic implants, embolic plugs, expandable implants, vascular plugs, embolic vascular endoprostheses, embolization beads, and embolic microspheres, of any suitable size, shape, and configuration. Further, prior to and/or subsequent to being coated, any of these embolic bodies (or any other suitable embolic body) can be coupled to or otherwise associated with one or more additionally thrombogenic components such as but not limited to strands, filaments, fibers including bundled fibers, windings, coils, particles, twisted elements, and/or meshes, whether such components are already coated or uncoated. As well, such additionally thrombogenic components can be incorporated into an embolic therapy device during and/or after the application of any coating layer to the device in accordance with the invention.

A helical coil such as the one depicted in Figure 1 may be constructed in any suitable manner and using any suitable equipment. Illustratively, a helical coil may be prepared by wrapping a suitable wire about a cylindrical or conical mandrel. In so doing, advantageous coil implants will be suitably configured to avoid substantially cutting, tearing, and/or causing other trauma to any surrounding soft tissues upon placement of the coils in the patient. Accordingly, any loose end of a helical wire coil may be placed axially through the core of the helix and/or such a wire end may be suitably bound to another part of the device, e.g., by heat, adhesives, and/or mechanical means. Further, any additional thrombogenic elements (e.g., particles, radial filaments, etc.) may be attached to portions of the coil by these and/or other suitable binding techniques, e.g., by tying or otherwise adhering them to the coil.

Embolic bodies which take the form of coils include but are not limited to helically wound coils, random wound coils, coils wound within coils, and other suitable coil configurations. Such coils may be formed with radiopaque metallic materials such as but not limited to those listed above. In some instances, several coils are placed at a given location within the vasculature, for example, within a vessel or within a space associated with a vessel such as an aneurysm sac, to more completely occlude, and in some cases, substantially or completely occlude, the flow of blood through the vessel or other space associated with the vessel. Thrombus formation on and around the coils further enhances the occlusive effect of the coils.

In some forms, embolic therapy devices of the disclosure are configured to resist unacceptable migration from the treatment site following implantation. Initially, device migration is inhibited, at least in part, by contact with tissues and/or other devices or materials at the implantation site, and then, after a period of time, the growth of new patient tissue (e.g., thrombus formation) into, on, and/or around the implanted device may help anchor the device. Illustratively, a device of the invention can be designed to conform to surrounding tissues at the implantation site and/or its design can take into account the type of tissue and the geometry at the implantation site and the ability of the implantation site tissues to conform around the device.

Although not necessary to broader aspects of the invention, in some embodiments, an embolic therapy device is configured to cause an acceptable amount of trauma to tissues at the treatment site upon deployment, which can serve to initiate a localized healing response effective to enhance the growth of new patient tissue at the treatment site. Additionally, certain inventive devices can be configured to embed within tissue at the implantation site, e.g., soft tissues surrounding a tumor, to inhibit the device from migrating from the site. However, any device capable of causing such traumas should be configured so as to not undesirably damage tissues at the treatment site.

Certain preferred embolic bodies have a degree of flexibility. For example, an embolic coil useful in some forms of the invention is formed with an elastic material that allows it to generally resume its original (relaxed) shape after being stretched or compressed. Of course, in some instances, such an elastic coil will be prevented from resuming its original shape upon deployment due to contact with other objects at the treatment site (e.g., patient tissues or other embolization devices packed into the vessel).

In some embodiments, an embolic coil is formed with an elastic material that allows it to attain a first, stretched configuration and a second, relaxed configuration. Illustratively, a helical coil can exhibit a generally linear, helical configuration when stretched and a comparatively compact, convoluted configuration when relaxed. This stretched configuration can be advantageous in some forms of the invention, for example, when a catheter having a particularly small diameter is needed to place the coil at the treatment site. Upon deployment from the catheter lumen and into the treatment site, such a coil can be allowed or caused to assume a relaxed configuration, which can enhance the occlusive characteristics of the emplaced coil.

Wire, when used in making an embolic body useful in some forms of the invention, can be of any suitable size, shape, and configuration, and can be formed with any suitable material(s), such as those listed above. Because addition of a coating material on a surface of an embolic wire can alter certain performance or other characteristics of the embolic wire, in accordance with the invention, a wire type can be selected to modulate one or more characteristics of the coated wire, for example, to provide a coated wire having a flexibility within a predetermined range. Of course, as discussed more thoroughly elsewhere herein, other factors such as but not limited to the type of coating material(s) selected, the number of coating layers applied, and the coating technique(s) utilized, can affect the performance and other characteristics of the coated device, and in this regard, different combinations of such factors can be developed through routine experimentation so as to provide a coated embolization device having suitable characteristics for a particular application.

The diameter of a piece of wire may or may not be constant along its length, and in certain aspects, is in the range of about 0.002 inches to about 0.100 inches, more typically in the range of about 0.005 to about 0.050 inches. In some forms, a suitable embolization coil has a primary coil, and potentially also a secondary coil. Such a primary coil can have a primary coil diameter, in a relaxed configuration, in the range of about 0.007 inches to about 0.120 inches, more typically from about 0.010 inches to about 0.030 inches. As well, the axial length of such an embolization coil, in a relaxed configuration, may vary, and is typically in the range of about 0.20 inches to about 50 inches, more typically from about 0.20 inches to about 40 inches. Such an embolic coil is typically wound to have between 2 and 100 turns per centimeter.

In one embodiment, an embolic coil is formed with wire having a diameter in the range of about 0.01 mm to about 0.1 mm, more typically from about 0.02 mm to about 0.05 mm. Such a coil can have a primary coil, and potentially also a secondary coil, wherein the primary coil diameter, in a relaxed configuration, is typically in the range of about 0.03 mm to about 0.140 mm, more typically in the range of about 0.05 mm to about 0.030 mm. As well, the axial length of the coil, in a relaxed configuration, may vary, and is typically in the range of about 30 cm to about 1000 cm, more typically from about 90 cm to about 300 cm. In certain aspects, the embolic coil is expandable so that in an unexpanded configuration, the wire is formed into a tightly-wound coil, having a diameter in the range of about 0.1 mm to about 1 mm, more typically from about 0.25 mm to about 0.5 mm, and a length in the range of about 2 mm to about 60 cm, more typically from about 25 mm to about 15 cm. The coil will typically have from about 20 turns to about 60,000 turns, more typically from about 1000 turns to about 6000 turns. In an expanded configuration (e.g., upon deployment), the wire forms a random structure larger in all dimensions than the initial, unexpanded coil, which can enhance the occlusive characteristics of the deployed coil.

In some forms, the present disclosure teaches bioactive materials (e.g. M1 macrophage promoting agents and/or M2 macrophage inhibiting agents) which have been coated on an embolic body. In certain embodiments the bioactive materials are immobilized by conjugation to a surface of the embolic body. In certain embodiments the bioactive materials are layered onto the surface of the embolic body. Any suitable method or technique for coating such bioactive agents onto the surface of an embolic body is within the scope of the present disclosure such that said bioactive agents are carried by said embolic body.

In certain embodiments, the present disclosure teaches embolic therapy devices which include a coating layer. In some forms, the coating layer includes one or more M1 macrophage promoting agents. In certain embodiments, the coating layer includes one or more M2 macrophage inhibiting agents. In some forms, the coating layer includes one or more M1 macrophage promoting agents, and one or more M2 macrophage inhibiting agents.

Embolic therapy devices of the present disclosure include one or more M1 macrophage promoting agents, M2 macrophage inhibiting agents, or a combination thereof carried by the embolic member. In some forms, the agents are conjugated to the embolic member. In certain preferred embodiments, embolic therapy devices of the present disclosure have a coating layer, which includes a (i.e., at least one) M1 macrophage promoting agent, M2 macrophage inhibiting agent, or a combination thereof (such a coating layer hereinafter sometimes referred to as a "Therapeutic Agent Release Layer" or "TA Release Layer"). Any embolic member discussed above or elsewhere herein may have a surface carrying a TA Release Layer as discussed herein, either as the sole coating carried by the surface carrying the TA Release Layer, or in combination with one or more additional coatings positioned underneath and/or overtop the TA Release Layers. As well, surfaces of the embolic member not carrying a TA Release Layer may optionally be bare (uncoated), or may carry one or more coatings that differ from the TA Release Layer.

The term "therapeutic effect" as used herein means an effect which induces, ameliorates or otherwise causes an improvement in the pathological symptoms, disease progression or physiological conditions associated with or resistance to succumbing to a disorder, for example revascularization, of an embolized tumor in a human or veterinary patient. The term, "therapeutically effective amount," as used with respect to a therapeutic agent means an amount of the therapeutic agent which imparts a therapeutic effect to the human or veterinary patient.

The M1 macrophage promoting agent and/or M2 macrophage inhibiting agent can be incorporated in the TA Release Layer at any suitable level. It will also be understood that the TA Release Layer may contain variations in the level of therapeutic agent in different regions of the coating either due to manufacturing variances or intentional design criteria. Thus, the present invention contemplates TA Release Layers in which the level of therapeutic agent(s) is substantially uniform over the entire area covered by the coating, or in which the level of therapeutic agent(s) differs substantially in one area covered by the TA Release Layer as compared to another area covered by the TA Release Layer

The M1 macrophage promoting agent and/or M2 macrophage inhibiting agent will typically be incorporated in the TA release layer, or otherwise carried by the embolic member, in a therapeutically effective amount. In this regard, it will be understood that in certain embodiments where the therapeutic agent is an M1 macrophage promoting agent, the M1 macrophage promoting agent may be incorporated in the coating in an amount that is effective to promote M1 macrophage recruitment and/or differentiation of M2 macrophages into M1 macrophages when the implantable medical device (e.g. embolic bead or coil) is deployed so as to deliver the therapeutic agent from the TA Release Layer to an area surrounding a tumor. Likewise, when the therapeutic agent is an M2 macrophage inhibiting agent, the M2 macrophage inhibiting agent may be incorporated in the coating in an amount that is effective to inhibit or block M2 macrophage promoters (e.g. IL-4, IL-10, IL-13, CSF-1) when the implantable medical device (e.g. embolic bead or coil) is deployed so as to deliver the therapeutic agent from the TA Release Layer to an area surrounding a tumor. As will be recognized, the level of a therapeutic agent that will be therapeutically effective will vary in accordance with the particular therapeutic agent in use, the implantable medical device in use, the implant site, the condition to be treated, the composition of the coating including the therapeutic agent, and other potential factors. Through routine experimentation in view of the disclosures herein the achievement of a therapeutically effective amount of the M1 macrophage promoting agent and/or M2 macrophage inhibiting agent will be within the purview of those of ordinary skill in the field.

It will be understood that in certain embodiments, the TA Release Layer can include ingredients in addition to the M1 macrophage promoting agent and/or the M2 macrophage inhibiting agent. For example, such other ingredients may be included to alter the physical, chemical and/or biologic properties of the TA Release Layer. Illustrative potential additional ingredients include, for example, ingredients that alter the release of the M1 macrophage promoting agent and/or the M2 macrophage inhibiting agent from the TA Release Layer and/or that alter the physical stability or adherence of the TA Release Layer to a surface of the embolic body or to another coating in turn adhered to the embolic body. The additional ingredient(s) in the TA Release Layer may for example be a biodurable polymer; a biodegradable polymer such as polylactic acid (PLA), polyglycolic acid (PGA), poly(lactic-co-glycolic acid) (PLGA), polyanhydride, polycaprolactone, polyhydroxybutyrate valerate, polyethylene glycol (PEG), or a mixture of any or all of these; a contrast agent, such as an iodinated contrast agent, e.g. iobitridol, iohexol, iomeprol, iopamidol, iopentol, iopromide, ioversol, ioxilan, iotrolan, iodixanol, ioxaglate; a molecule having a hydrophilic part and a hydrophobic part, such as a surfactant (e.g. a nonionic surfactant such as a polysorbate surfactant); or one or more water-soluble therapeutic agents; urea; butyryl-tri-hexyl citrate; or a mixture of any or all of these. An additional ingredient may also comprise an immune-stimulating agent including, for example, chitosan.

Any of a wide variety of coating patterns may be used to constitute a material coat on the embolic body, or upon additional coating layers. The TA Release Layer can be directly adhered to a surface of an embolic body and provide an outermost surface over the embolic body, and/or to constitute the entirety of the overall material coat on the embolic body. In other embodiments, an overall material coat adhered to the embolic body can include one or more different coatings positioned underneath the TA Release Layer (e.g. as in a polymeric or other primer coating, or a different therapeutic agent coating, adhered directly to the surface of the medical device), one or more different coatings positioned overtop the TA Release Layer (e.g. as in a polymeric or other protective or diffusion barrier coating), or both. As well, there may be one or more different coatings adjacent the TA Release Layer, and/or multiple TA Release Layers may be carried by the embolic body at locations discrete from one another. The TA Release Layer(s) may occur in an aperture(s) such as a well(s), groove(s) or hole(s) defined in the embolic body or may partially coat or completely coat the embolic body or a given surface (e.g. inner, outer or side surface) of the embolic body. These and other overall device coating arrangements can be utilized.

The TA Release Layer can be carried by any suitable surface of the embolic body. The TA Release Layer can be carried by, and in some embodiments only by, a surface or surfaces of the embolic therapy device configured for contact with patient tissue when the device is implanted. For example, in some embodiments the TA Release Layer is carried by a surface of an embolic coil which is configured for contact with a wall of a vessel when the embolic coil is introduced into the vessel. On the other hand, in certain embodiments, the TA Release Layer is carried by an inner surface of an embolic coil which is not configured for contact with the vessel wall.

A first TA Release Layer can be present in combination with another layer including the M1 macrophage promoting agent and/or the M2 macrophage inhibiting agent without additional ingredients, or with a second TA Release Layer including a lower relative concentration of M1 macrophage promoting agent and/or the M2 macrophage inhibiting agent than the first TA Release Layer, such that the other layer or the second TA Release Layer releases the M1 macrophage promoting agent and/or the M2 macrophage inhibiting agent at a rate slower than the first TA Release Layer. For example, a layer including the M1 macrophage promoting agent and/or the M2 macrophage inhibiting agent can be at least partially overcoated, or undercoated, with a TA Release Layer including the same or another therapeutic agent. In one embodiment, an embolization therapy product can include a relatively faster releasing TA Release Layer which at least partially over coats a layer which releases M1 macrophage promoting agent and/or the M2 macrophage inhibiting agent at a relatively slower rate. This configuration can allow for the quick delivery of the M1 macrophage promoting agent(s) and/or the M2 macrophage inhibiting agent(s) from the TA Release Layer followed by a more gradual delivery of the M1 macrophage promoting agent(s) and/or the M2 macrophage inhibiting agent(s) from the relatively slower dissolving layer. The two layers can be separated by one or more layers. Such a configuration can be used in combination with the coating patterns discussed above.

In some aspects, the present disclosure provides a method of forming a coated embolization device, such method comprising providing an embolic body as described above and layering a bioactive material on the surface of the embolic body. In some forms, the bioactive material comprises one or more M1 macrophage promoting agents, M2 macrophage inhibiting agents, or a combination thereof. The TA Release Layer and any other coating layers present can be incorporated as a part of the embolization product by any suitable method. The TA Release Layer and any other coating layer can be formed on a surface of the embolic therapy device. For example, the TA Release Layer or other coating layer(s) can be formed by a method that includes dipping, spraying, showering, dripping, or otherwise applying a medium containing the coating ingredients, and optionally a substance such as a solvent can be removed from the medium to leave the coating adhered to the implantable medical device. Spray coating is one preferred form of applying the coating materials to the surface of the embolic body, and in particular embodiments ultrasonic spray coating or pressure spray coating will be utilized. During spray coating or other coating operations, the embolic body can be moved relative to a sprayer or other applicator of the coating ingredients. This can occur by moving the embolic body (including, for example, rotating the device or at least the portion to be coated), moving the sprayer or other applicator, or both. Multiple application passes or steps will typically be utilized to increase the thickness of the TA Release Layer or other coating layer(s) and control the levels of the M1 macrophage promoting agent(s) and/or the M2 macrophage inhibiting agent(s), or other ingredients applied to the embolic body. In spray or other application processes, areas of the embolic body adjacent to areas desired for coating can optionally be masked to prevent the application of coating materials to the masked areas, and /or portions of applied coating materials can be removed to selectively leave a TA Release Layer or other coating in a desired region or regions of the embolization product.

The M1 macrophage promoting agent(s) and/or the M2 macrophage inhibiting agent(s) (and potentially other ingredients) can be combined in a liquid to form a coating medium to be used in the formation of the TA Release Layer on the embolic body. This combination can be in the form of a liquid emulsion, suspension, solution, or any other suitable flowable form. Coating mediums provided as solutions are preferred.

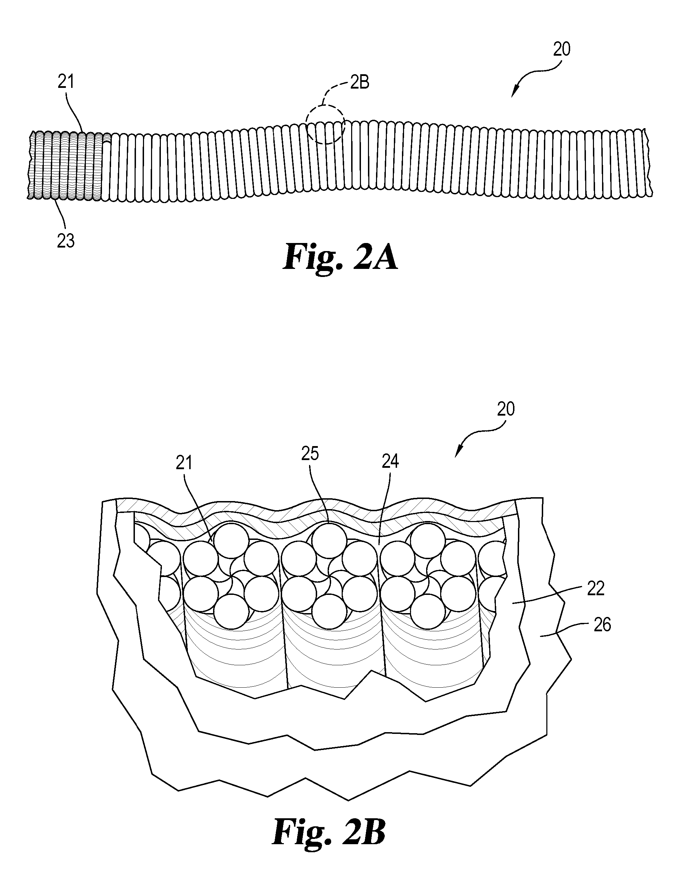

With reference now to Figures 2A and 2B, shown is a perspective view of another illustrative embolic therapy device 20 of the disclosure. The embolic therapy device 20 includes an embolic body 21 a first coating layer 22 including one or more M1 macrophage promoting agents, M2 macrophage inhibiting agents, or a combination thereof and a second coating layer 26. The second coating layer 26 may also include one or more M1 macrophage promoting agents, M2 macrophage inhibiting agents, or a combination thereof. The embolic body 21, which is illustrated in the form of a tightly wound yet flexible coil may be comprised of any suitable biocompatible material. It should be appreciated that when an embolic device of the present disclosure carries more than one coating layer, such layers may comprise, for example, alternate layer thicknesses, alternate relative agent concentrations, and/or alternate ingredients. In this embodiment, the embolic body 21 is shown having an uncoated portion 23 for illustrative purposes.

Further in this regard, a coating layer of the disclosure need not uniformly coat the embolic body surfaces which it coats. In accordance with certain aspects of the present disclosure, for example as shown in Figures 2A and 2B, relatively more coating material may accumulate and become immobilized and/or stabilized in spaces **24** between individual turns of the coil compared to other spacers surrounding the coil turn, e.g. those adjacent to the top, outer surfaces **25** of the turns.

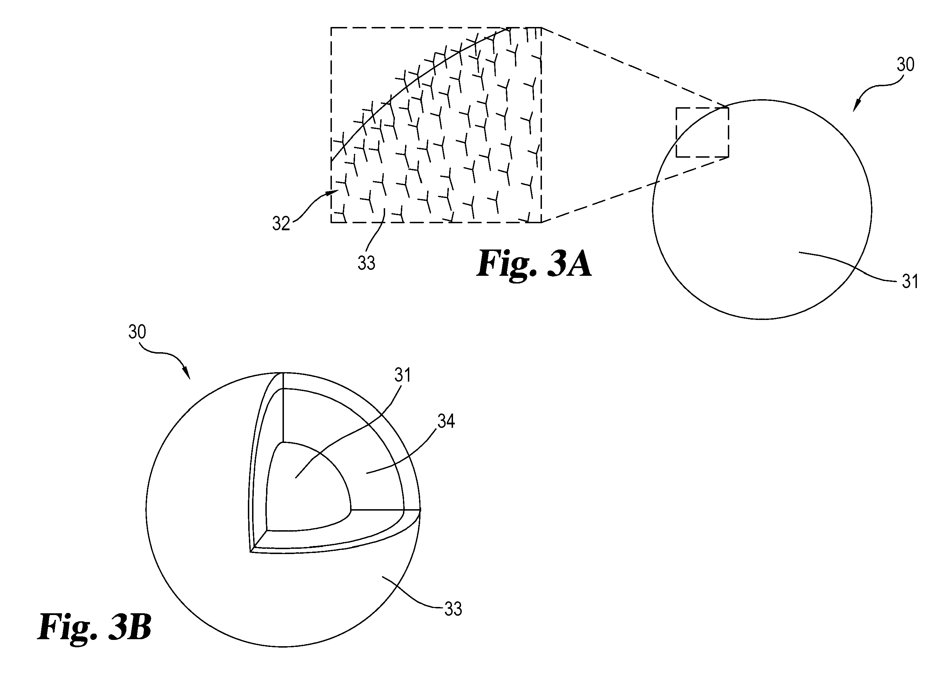

With reference to FIG. **3A****,** shown is a perspective view of another illustrative embolic therapy device **30** of the disclosure. The embolic therapy device **30** includes an embolic body **31** with one or more M1 macrophage promoting agents, M2 macrophage inhibiting agents, or a combination thereof **32** conjugated to the outer surface **33** of embolic body **31.** In some forms, outer surface **33** is continuous with the rest of embolic body **31,** that is outer surface **33** is composed of substantially the same materials as embolic body **31.** In certain embodiments, as illustrated in FIG **3B** which provides a cutaway perspective view of an embolic body of the present disclosure, outer surface **33** comprises a coating layer adhered to embolic body **31,** in accordance with such embodiments, the coating layer may further include bioactive agents such as one or more M1 macrophage promoting agents, M2 macrophage inhibiting agents, or a combination thereof. Certain embodiments include one or more additional coating layers **34.** Any of one or more coating layers **34** and/or outer surface **33** may include one or more M1 macrophage promoting agents, M2 macrophage inhibiting agents, or a combination thereof. The embolic body **31,** which is illustrated in the form of an embolic bead or microsphere, may be composed of any suitable biocompatible material.

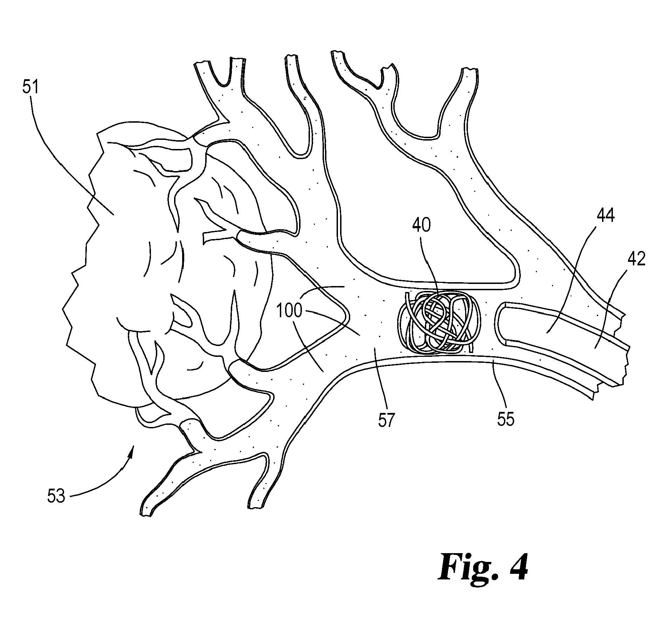

With reference to FIG. **4****,** shown is a cutaway view of vasculature **55** surrounding a tumor **51.** Supply vessels **53** supply blood to tumor **51.** In accordance with certain inventive variants, embolic therapy devices **40** are utilized to substantially block, occlude, or inhibit blood supply to target tumor **51.** As detailed above, embolic therapy devices **40** of the present disclosure comprise one or more therapeutic agents **100,** for example, M1 macrophage promoting agents, M2 macrophage inhibiting agents, or a combination thereof. Such devices are used, for example, to inhibit blood flow and to deliver and release one or more M1 macrophage promoting agents, M2 macrophage inhibiting agents, or a combination thereof to the area surrounding a target tumor. Certain inventive methods include advancing a delivery device **42,** for example a catheter, or injection needle, to a deployment site **57** near tumor **51,** and implanting one or more embolization devices **40.** In some forms, delivery device **42** includes a lumen **44.**

All publications and patent applications cited in this specification are herein incorporated by reference as if each individual publication or patent application were specifically and individually indicated to be incorporated by reference. Further, any theory, mechanism of operation, proof, or finding stated herein is meant to further enhance understanding of the present invention, and is not intended to limit the present invention in any way to such theory, mechanism of operation, proof, or finding. While the invention has been illustrated and described in detail in the drawings and foregoing description, the same is to be considered as illustrative and not restrictive in character, it being understood that only selected embodiments have been shown and described and that all equivalents, changes, and modifications that come within the spirit of the inventions as defined herein or by the following claims are desired to be protected. It will be evident from the specification that aspects or features discussed in one context or embodiment will be applicable in other contexts or embodiments.

Preferred embodiments of the present invention are described below and are referred to as embodiments E1 to E23:
E1. An embolic therapy device comprising:
   an embolic body; and
   one or more M1 macrophage promoting agents, or one or more M2 macrophage inhibiting agents, wherein said one or more agents are carried by said embolic body.
E2. The embolic therapy device of E1, further comprising:
   one or more coating layers carried by a surface of said embolic body, and including said one or more agents, said coating layers effective to release said agents.
E3. The embolic therapy device of E1, wherein:
   said M1 promoting agent comprises histidine-rich glycoprotein.
E4. The embolic therapy device of E1, wherein:
   said M1 promoting agent comprises 17β-estradiol.
E5. The embolic therapy device of E1, wherein:
   said M1 promoting agent comprises interferon-gamma.
E6. The embolic therapy device of E1, wherein:
   said M1 promoting agent comprises a lipopolysaccharide.
E7. The embolic therapy device of E1, wherein:
   said M1 promoting agent comprises iron.
E8. The embolic therapy device of E1, wherein:
   said M1 promoting agent comprises an anti-CD47 blocking antibody.
E9. The embolic therapy device of E1, wherein:
   said M2 inhibiting agent is effective to inhibit interleukin-4.
E10. The embolic therapy device of E 1, wherein:
   said M2 inhibiting agent is effective to inhibit interleukin-13.
E11. The embolic therapy device of E1, wherein:
   said M2 inhibiting agent is effective to inhibit interleukin-10.
E12. The embolic therapy device of E1, wherein:
   said M2 inhibiting agent is effective to inhibit colony stimulating factor 1.
E13. The embolic therapy device of E1, wherein:
   said embolic body comprises an embolic coil.
E14. The embolic therapy device of E1, wherein:
   said embolic body comprises an embolic bead.
E15. The embolic therapy device of E1, further comprising:
   an immune stimulating compound, carried by said embolic body.
E16. The embolic therapy device of E15, wherein:
   said immune stimulating compound comprises chitosan.
E17. A method of forming a coated embolization device, said method comprising:
   coating a bioactive material on a surface of an embolic body, said bioactive material comprising one or more M1 macrophage promoting agents, M2 macrophage inhibiting agents, or a combination thereof.
E18. The method of E17, wherein the embolic body is an embolic coil.
E19. The method of E17, wherein the embolic body is an embolic bead.
E20. A method of treating a patient, comprising:
   implanting in the patient one or more embolization therapy devices according to any one of E1-16.
E21. The method of E20, wherein said one or more agents is effective to: stimulate the recruitment of M1 macrophages, stimulate monocyte development into M1 macrophages, stimulate differentiation of M2 macrophages into M1 macrophages, inhibit the recruitment of M2 macrophages, inhibit monocyte development into M2 macrophages, and/or inhibit differentiation of M1 macrophages into M2 macrophages.
E22. An M1 macrophage promoting agent for treatment of cancerous tumors, wherein said M1 macrophage promoting agent is carried on an embolic member. E23. An M2 macrophage inhibiting agent for treatment of cancerous tumors, wherein said M2 macrophage inhibiting agent is carried on an embolic member.

## Claims

1. An embolic therapy device comprising:
an embolic body; and
one or more agents selected from M1 macrophage promoting agents and M2 macrophage inhibiting agents, wherein said one or more agents are carried by said embolic body.

2. The embolic therapy device of claim 1, further comprising:
one or more coating layers carried by a surface of said embolic body, and including said one or more agents, said coating layers effective to release said agents.

3. The embolic therapy device of claim 1 or claim 2, wherein:
said one or more agents include an M1 macrophage promoting agent selected from histidine-rich glycoprotein, 17β-estradiol, interferon-gamma, a lipopolysaccharide, iron and an anti-CD47 blocking antibody.

4. The embolic therapy device of any preceding claim, wherein:
said one or more agents include an M2 macrophage inhibiting agent which is effective to inhibit one or more of interleukin-4, interleukin-13, interleukin-10 and colony stimulating factor 1.

5. The embolic therapy device of any preceding claim, wherein:
said embolic body comprises an embolic coil.

6. The embolic therapy device of any of claims 1 to 4, wherein:
said embolic body comprises an embolic bead.

7. The embolic therapy device of any preceding claim, further comprising:
an immune stimulating compound, carried by said embolic body.

8. The embolic therapy device of claim 7, wherein:
said immune stimulating compound comprises chitosan.

9. A method of forming a coated embolization device, said method comprising:
coating a bioactive material on a surface of an embolic body, said bioactive material comprising one or more M1 macrophage promoting agents, M2 macrophage inhibiting agents, or a combination thereof.

10. The method of claim 9, wherein the embolic body is an embolic coil.

11. The method of claim 9, wherein the embolic body is an embolic bead.

12. An embolization therapy device of any of claims 1 to 8 for use in a method of treating a patient, the method comprising implanting the embolization therapy device in the patient.

13. The embolization therapy device for use of claim 12, wherein the one or more agents are effective to: stimulate the recruitment of M1 macrophages, stimulate monocyte development into M1 macrophages, stimulate differentiation of M2 macrophages into M1 macrophages, inhibit the recruitment of M2 macrophages, inhibit monocyte development into M2 macrophages, and/or inhibit differentiation of M1 macrophages into M2 macrophages.

14. An M1 macrophage promoting agent suitable for treating cancerous tumors, wherein said M1 macrophage promoting agent is carried on an embolic member.

15. An M2 macrophage inhibiting agent suitable for treating cancerous tumors, wherein said M2 macrophage inhibiting agent is carried on an embolic member.
